(19) Europäisches Patentamt
European Patent Office
Office européen des brevets

(11) Publication number: **0 447 345 A2**

(12) **EUROPEAN PATENT APPLICATION**

(21) Application number: **91500014.5**

(51) Int. Cl.[5]: **C07D 221/20,** A61K 31/435

(22) Date of filing: **11.02.91**

The title of the invention has been amended (Guidelines for Examination in the EPO, A-III, 7.3).

(30) Priority: **13.02.90 ES 9000421**

(43) Date of publication of application: **18.09.91 Bulletin 91/38**

(84) Designated Contracting States: **AT BE CH DE DK FR GB GR IT LI LU NL SE**

(71) Applicant: **FABRICA ESPANOLA DE PRODUCTOS QUIMICOS Y FARMACEUTICOS, S.A. (FAES)**
**c/ Maximo Aguirre, 14**
**E-48940 Leioa-Lamiaco (Vizcaya) (ES)**

(72) Inventor: **Orjales-Venero, Aurelio**
**Po del Puerto, 24**
**Neguri (Vizcaya) (ES)**
Inventor: **Rodes-Solanes, Rosa**
**C/ Bizkerre, 30- 1oC**
**E-48990 Guecho (Vizcaya) (ES)**

(74) Representative: **Isern-Cuyas, Maria Luisa**
**Paseo de la Castellana 131, Bajo C**
**E-28046 Madrid (ES)**

(54) **New Azaspyro [4.5] decane-7,9-dione derivatives.**

(57) THE INVENTION RELATES NEW AZASPYRO[4.5]DECANE-7,9-DIONE DERIVATIVES of general formula (I):

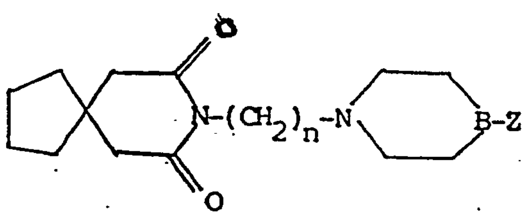

(I)

where n is 2 or 4; B can be a nitrogen atom or a -CH-group; Z can be the radical (pyrimidine 2 yl)amino, the radical 3-trifluoromethylphenyl and the radical benzimidazole-2-yl, substituted in position 1 with short chain alkyl radicals or with a phenylmethyl radical, with or without substituents in the ring.

EP 0 447 345 A2

## NEW AZASPYRO[4.5]DECANE-7,9-DIONE DERIVATIVES

The present invention relates new azaspyro[4.5]decane-7,9-dione derivatives, and pharmaceutically acceptable salts thereof, of pharmacological value for the central nervous system and with the following linkage:

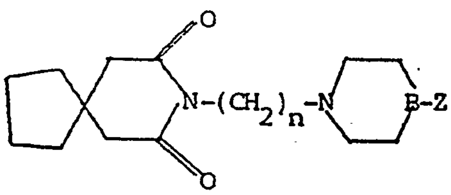

(I)

where n is 2 or 4; B can be a nitrogen atom or a -CH- group; Z can be the radical ( pyrimidine 2 yl)amino, the radical 3-trifluoromethylphenyl and the radical benzimidazole-2-yl, substituted in position 1 with short chain alkyl radicals or with a phenylmethyl radical, with or without substituents in the ring.

Many azaspyroalkanedione derivatives are known to be of pharmacological value for the central nervous system. Compounds of this kind were prepared and described in works and patents as psychotropics, tranquillizers, anxiolytics and so forth. Among all the compounds described, especially interesting are the azaspyrodecanediones similar to the buspyrone molecule, described by Wu Y H in US Patent No. 3717634 (1973); US Patent No. 3907801 (1976), and with the following general linkage formula:

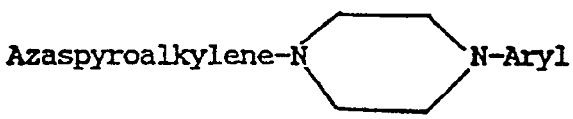

where aryl can be a phenyl group or a heterocycle (pyrimidine in the case of buspyrone) with or without substituents. Such molecules are pharmacologically interesting because of their anxiolytic activity since they are able to interact with $5HT_{1A}$ receptors, these latter being especially linked to states of anxiety.

A preliminary study determined the affinity of the compounds described herein by the $5HT_{1A}$ receptors by means of the technique originally described by Gozlan et al (Nature 305, 140-2 (1983)) for radioligand union and displacement in a tissue obtained from the front cortex of a rat and using $[^3H]$ 8-0h-DPAT as ligand. The compounds studied show a degree of affinity by such receptors similar to that of the buspyrone molecule ($K_I$ = 1.99 x 10).

Antihistaminic $H_1$ activity was also discovered, in tests made using the technique described by Lefevre et al (C.R. Soc. Biol. 156, 183, 1962), and at levels similar to those of the reference substances used, terphenadine and astemizole.

The following examples illustrate the invention, without being fully comprehensive thereof.

### Example 1

Preparation of 8-[4-[4-[(3-trifluoromethyl)phenyl]-1-piperazinyl]butyl]-8-azaspyro[4.5]decane-7,9-dione.

A mixture containing 1.5 grammes (5 mmoles) of (4-aminobutyl)-4-(3-trifluoromethyl)phenyl piperazine, 0.83 grammes (5 mmoles) of 3,3-tetramethyleneglutaric anhydride and 20 ml. of pyridine were refluxed for 20 hours. It was then boiled down to vacuum and the residuum dissolved in $CH_2Cl_2$, washed with water and the organic phase dried on anhydrous $Na_2SO_4$. Boiling down again to vacuum gave a thick oil that solidified with hexane/ether, forming a white solid that was filtered to yield 1.5 grammes (3.3 mmoles, 66% yield) of a product with a melting point of 68-70°C. Nuclear-magnetic-resonance and infrared spectroscopy data confirmed the 8-[4-[4-[(3-trifluoromethyl)phenyl]-1-piperazinyl]butyl]-8-azaspyro[4.5]decane-7,9-dione structure.

Example 2

Preparation of 8-[4-[4-(1-ethyl-benzimidazole-2-yl)1-piperazinyl]butyl]-8-azaspyro[4.5]decane-7,9-dione fumarate.

A solution of 1.5 grammes (5 mmoles) of 1-ethyl-2-[4-(4-aminobutyl)-1-piperazinyl]benzimidazole and 0.83 grammes (5 mmoles) of 3,3-tetramethyleneglutaric anhydride in 20 ml. of toluene were refluxed in Dean-Stark gear for 5 hours. After the reaction time was over, the solvent was boiled down at low pressure and the residuum suspended in water and drawn off with $CH_2Cl_2$. The organic phase was washed with water and dried on anhydrous $Na_2SO_4$. The solvent was then evaporated to give 1.3 grammes of yellow oil. This oil was dissolved in ethanol, and 0.34 grammes of fumaric acid dissolved in ethanol were added, refluxing for 15 minutes. The solvent was boiled down and the residuum crystallised in ethanol/ether to give 1.13 grammes (2 mmoles: yield 40%) of a white solid with a melting point of 173-174°C, the nuclear-nuclear-magnetic-resonance and infrared spectroscopy data thereof confirming the 8-[4-[4-(1-ethyl-benzimidazole-2-yl)-1-piperazinyl]butyl]-8-azaspyro[4.5]decane-7,9-dione fumarate structure.

## Claims

1. - NEW AZASPYRO[4.5]DECANE-7,9-DIONE DERIVATIVES of general formula (I):

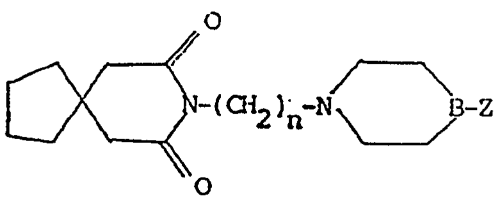

(I)

and their pharmaceutically acceptable salts, where B is a nitrogen atom or a -CH- group, n can be either 2 or 4, and Z can be the radical (pyrimidine-2-yl)amino, the radical 3-trifluoromethylphenyl and the radical benzimidazole-2-yl, substituted in position 1 with short chain alkyl radicals or with a phenylmethyl radical, with the phenyl group substituted in position 4 by an F atom.

2. - NEW AZASPYRO[4.5]DECANE-7,9-DIONE DERIVATIVES according to the previous claim, characterised in being:

8-[4-[4-(1-ethyl-benzimidazole-2-yl)-1-piperazinyl]butyl]-8-azaspyro[4.5]decane-7,9-dione; 8-[4-[4-(1-methyl-benzimidazole-2-yl)-1-piperazinyl]butyl]-8-azaspyro[4.5]decane-7,9-dione; 8-[4-[4-[(pyrimidine-2-yl)amino]-1-piperidinyl]butyl]-8-azaspyro[4.5]decane-7,9-dione; 8-[4-[4-[(1-phenylmethyl)-benzimidazole-2-yl]-1-piperazinyl]butyl]-8-azaspyro[4.5]decane-7,9-dione; 8-[4-[4-[1-(4′-fluorophenylmethyl)benzimidazole-2-yl]-1-piperazinyl]butyl]-8-azaspyro[4.5]decane-7,9-dione; 8-[2-[4-(3-trifluoromethylphenyl)-1-piperazinyl] ethyl]-8-azaspyro[4.5]decane-7,9-dione and 8-[4-[4-(3- trifluoromethylphenyl)-1-piperazinyl]butyl]-8-azaspyro[4.5]decane-7,9-dione, and their addition salts with pharmaceutically acceptable organic and inorganic acids, preferably hydrochloric and fumaric acids.

## Claims for the following Contracting States: AT and GR

3. - A process for obtaining compounds of general formula (I):

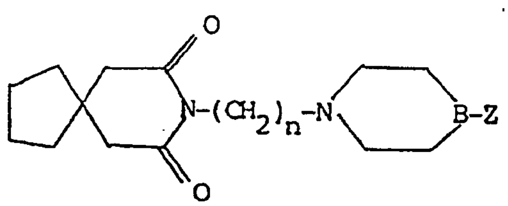

(I)

and their pharmaceutically acceptable salts, where B is a nitrogen atom or a -CH- group, n can be either 2 or 4, and Z can be the radical (pyrimidine-2-yl)amino, the radical 3-trifluoromethylphenyl and the radical benzimidazole-2-yl, substituted in position 1 with short chain alkyl radicals or with a phenylmethyl radical, with the phenyl group substituted in position 4 by an F atom, characterised in reacting 3,3-tetramethyleneglutaric anhydride within a suitable solvent, preferably pyridine, toluene and butanol, and at a suitable temperature ranging between 80 and 140°C, preferably at the boiling point of the solvent used, with a primary amine of general formula (II):

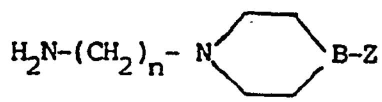

(II)

where Z, B and n have the aforesaid values.

**4.** - A process according to the previous claim, characterised in that the azaspyro[4.5]decane-7,9-dione derivatives synthesised are: 8-[4-[4-(1-ethyl-benzimidazole-2-yl)-1-piperazinyl]butyl]-8-azaspyro[4-5]decane-7,9-dione; 8-[4-(4-(1-methyl-benzimidazole-2-yl)-1-piperazinyl]butyl]-8-azaspyro[4-5]decane-7,9-dione; 8-[4-[4-[(pyrimidine-2-yl)amino]-1-piperidinyl)butyl)-8-azaspyro[4-5]decane-7,9-dione; 8-[4-[4-[(1-phenylmethyl)-benzimidazole-2-yl)-1-piperazinyl)butyl)-8-azaspyro[4.5]decane-7,9-dione; 8-[4-[4-[1-(4'-fluorophenylmethyl)benzimidazole-2-yl)- 1-piperazinyl]butyl]-8-azaspyro[4.5]decane-7,9-dione; 8-[2-[4-(3-trifluoromethylphenyl)-1-piperazinyl]ethyl]-8-azaspyro[4.5]decane-7,9-dione and 8-[4-[4-(3-trifluoromethylphenyl)-1-piperazinyl]butyl]-8-azaspyro[4.5]decane-7,9-dione, and their addition salts with pharmaceutically acceptable organic and inorganic acids, preferably hydrochloric and fumaric acids.

**5.** - A process for obtaining new azaspyro[4.5]decane-7,9-dione derivatives.